# EUROPÄISCHE PATENTANMELDUNG

(11) **EP 0 968 701 A2**
(43) Veröffentlichungstag der Anmeldung: **05.01.2000**
(21) Anmeldenummer: 99112664.0
(22) Anmeldetag: 02.07.1999
(51) Int. Cl.: A61K 6/083

(54) **Mikro- und/oder mesoporöse zeolithische Materialien als Füllkomponenten in Dentalkompositen**

(30) Priorität: 03.07.1998 DE 19829870
(71) Anmelder: Degussa-Hüls Aktiengesellschaft, 60287 Frankfurt am Main (DE)
(72) Erfinder: Möller, Karin, Dr., West Lafayette, IN 47906 (US); Bein, Thomas, Prof.Dr., West Lafayette, IN 47906 (US); Fischer, Reinhard, Prof.Dr., 28357 Bremen (DE); Rentsch, Harald, Dr., 63457 Hanau (DE)

(57) **Zusammenfassung**

Die vorliegende Erfindung richtet sich auf einen Dentalwerkstoff, der als Füllkomponente zeolithische mikro- und oder/mesoporöse zeolithische Materialien enthält.

Dadurch, daß mikro- und oder/mesoporöse zeolithische Materialien als Füllkomponente in Dentalkompositen auf Kunststoffbasis verwendet werden, ergibt sich eine verbesserte Langzeitstabilität gegenüber vergleichbaren Dentalkompositen des Standes der Technik.

## Beschreibung

Die vorliegende Erfindung beschreibt einen polymerisierbaren Dentalwerkstoff sowie ein Verfahren zu dessen Herstellung. Die Erfindung richtet sich weiterhin auf die Verwendung von bestimmten, mit polymerisierbaren, ethylenisch ungesättigten Monomeren oder mit deren polymerisierter Form und/oder durch Silankopplung mit weiteren geeigneten Molekülen beladenen mikro- und/oder mesoporösen zeolithischen Füllstoffen (A) in verbesserten, polymerisierbaren Dentalwerkstoffen.

Insbesondere richtet sich die Erfindung auf Dentalwerkstoffe auf Basis von polymerisierbaren, ethylenisch ungesättigten Monomeren als Bindemittel, eines Katalysators für die Kalt-, Heiß- und/oder Photopolymerisation und bezogen auf den Dentalwerkstoff 1-95 Gew.-% eines zeolithischen Füllstoffs (A) mit polymerisierbaren, ethylenisch ungesättigten Monomeren oder mit deren polymerisierter Form und/oder durch Silankopplung mit weiteren geeigneten Molekülen beladener zeolithischer Mikro- und/oder Mesoporenstruktur sowie bezogen auf den Dentalwerkstoff 0-95 Gew-% von weiteren Füllstoffen (B).

Auf Grund der möglichen Gesundheitsgefahren durch quecksilberhaltige Materialien (Amalgame) im Bereich der Zahnrestauration ist man verstärkt auf der Suche nach neuen quecksilberfreien Zubereitungen für diesen Anwendungszweck.

Die EP-A 0 530 926 offenbart Dentalmassen aus einem polymerisierbaren Monomer und einem anorganischen Füller, der zu 20 bis 80 Gew.-% aus sphärischen anorganischen Oxidpartikeln mit einer durchschnittlichen Teilchengröße zwischen 1,0 und 5,0 µm und zu 80 - 20 Gew.-% aus sphärischen anorganischen Oxidpartikeln mit einer Teilchengröße im Bereich von mindestens 0,05 µm und weniger als 1,0 µm besteht, wobei wenigstens 5 Gew.-% der letztgenannten Komponente im Bereich von 0,05 bis 0,2 µm liegen. Bei den anorganischen Partikeln handelt es sich ausschließlich um kugelförmige Partikel anorganischer Oxide des Siliciums, Zirkoniums, Aluminiums und Titans oder Mischoxide von Metallen aus der I - IV Hauptgruppe des Periodensystems mit Silicium. Die kugelförmigen Partikel werden z. B. durch hydrolytische Polymerisation von Alkoxysilanen hergestellt und können z. B. auch mit γ-Methacryloxypropyltrimethoxysilan oberflächenbehandelt werden.

In der DE 196 15 763 werden mit Monomeren beladene amorphe Siliziumdioxid-Gläser in Dentalkompositen beschrieben.

Bei den in der EP 48 681 beschriebenen befüllten anorganischen porösen Teilchen handelt es sich ebenfalls um auf amorphen Gläsern beruhenden Füllstoffen.

Diese und andere derzeit erhältliche Zahnrestaurationsmaterialien sind den Amalgamen in mehrfacher Hinsicht unterlegen:
- die Füllmaterialien sind oft zu weich, um im Kaubereich der Zähne verwendet zu werden
- die normalerweise in die Polymermischungen einpolymerisierten nichtporösen Materialien, wie z. B. Glaspartikel, müssen mit speziellen Kopplungsreagenzien überzogen werden und sind oftmals anfällig für hydrolytische Spaltungen zwischen Polymermaterial und Füllstoff
- die Füllmaterialien sind aufwendig in der Herstellung.

Aufgabe der vorliegenden Erfindung war es deshalb, einen Dentalwerkstoff anzugeben, der im Hinblick auf die Festigkeit und die hydrolytische Spaltbarkeit der Bindung zwischen anorganischem Füllstoff und Polymerumgebung bei gleichbleibend geringer Volumenkontraktion bei der Aushärtung den Dentalmaterialien des Standes der Technik auch hinsichtlich der leichten Herstellung überlegen ist.

Aufgabe der Erfindung ist auch die Angabe von weiteren Dentalwerkstoffen mit porösen mit Monomeren befüllbaren zeolithischen Füllstoffen.

Aufgabe der Erfindung ist weiterhin die Angabe von Verwendungen von bestimmten mikro- bzw. mesoporösen zeolithischen Füllstoffen bzw. die Angabe eines Herstellverfahrens für den neuen Dentalwerkstoff.

Die Lösung dieser sowie weiterer im einzelnen nicht näher genannter Aufgaben, die sich jedoch für den Fachmann in naheliegender Weise aus dem Stand der Technik ergeben, gelingt durch einen Dentalwerkstoff der eingangs erwähnten Gattung, welcher die Merkmale des kennzeichnenden Teils des Anspruches 1 aufweist. Zweckmäßige Ausgestaltungen des erfindungsgemäßen Dentalwerkstoffs werden in den von Anspruch 1 abhängigen Ansprüchen unter Schutz gestellt. Hinsichtlich des Verfahrens und der Verwendung bieten Ansprüche 8 bis 12 einen Vorschlag für die Lösung der oben angegebenen Probleme an. Eine bevorzugte Verwendung wird in Anspruch 13 angegeben.

Dadurch, daß der Dentalwerkstoff auf Basis von polymerisierbaren, ethylenisch ungesättigten Monomeren als Bindemittel, eines Katalysators für die Kalt-, Heiß- und/oder Photopolymerisation und bezogen auf den Dentalwerkstoff 1-95 Gew.-% eines anorganischen kristallinen zeolithischen Füllstoffs (A) sowie bezogen auf den Dentalwerkstoff 0-95 Gew.-% von weiteren Füllstoffen (B) enthält, wobei der zeolithische Füllstoff (A) mit polymerisierbaren, ethylenisch ungesättigten Monomeren oder mit deren polymerisierter Form und/oder durch Silankopplung mit weiteren geeigneten Molekülen beladene zeolithische Mikro- und/oder Mesoporen aufweist, gelingt es, einen polymerisierbaren Dentalwerkstoff mit im auspolymerisierten Zustand verbesserter Festigkeit, geringer Volumenkontraktion bei der Aushärtung und äußerst geringer hydrolytischer Spaltbarkeit zwischen Füllstoff und Polymer in relativ einfacher Weise bereitzustellen.

Bevorzugt wird ein Dentalwerkstoff eingesetzt, bei dem der Füllstoff (A) ein Zeolith ist der vor Befüllung mit Monomeren dealuminiert wurde.

Bei Dentalfüllungen liegt im auspolymerisierten Zustand das Verhältnis der Gew.-% von Polymer zu Füllstoff (A) zu Füllstoffen (B) im Verhältnisbereich von 10-40 zu 1-20 zu 89-40 liegt, bevorzugt zwischen 15-30 zu 3-15 zu 82-55 (in Summe 100 Gew.-%).

Bei dem erfindungsgemäßen Dentalwerkstoff kann man den Füllstoff (A) zusätzlich vor der Monomerimbibierung mit röntgenopaken Ionen durch Ionenaustausch beladen.

Demgegenüber läßt sich der Füllstoff (A) zusätzlich vor der Monomerbeladung durch gemeinsame Fällung in den Poren und/oder im Kristallgitter mit röntgenopaken Verbindungen beladen.

Weiterhin können die Poren des Füllstoffs (A) zur Aufnahme von bacteriziden Mitteln dienen.

Die Füllstoffe (A) und/oder (B) können darüber hinaus auch durch weitere chemische Modifizierung der Öberfläche verbessert werden.

Dentalwerkstoff bezeichnet im Rahmen der Erfindung Materialien für die Zahnrestauration, wie Zahnfüllung, Inlays oder Onlays, Befestigungszemente, Glasionomerzemente, Kompomere, Verblendmaterialien für Kronen und Brücken, Materialien für künstliche Zähne, in Dentinbondings, Unterfüllmaterialien, Wurzelfüllmaterialien oder sonstige Materialien für die prothetische, konservierende und präventive Zahnheilkunde. Insbesondere fallen unter den Begriff Dentalwerkstoff auch Komposits für zahnmedizinische und zahntechnische Einsatzzwecke, Versieglermaterialien, selbsthärtende Komposits, Stumpfaufbaumaterialien, Verblendkunststoffe, hoch- und normalgefüllte Dualzemente sowie normalgefüllte fluoridhaltige Zahnlacke.

Als Bindemittel kommen für den Dentalwerkstoff alle diejenigen Bindemittel auf Basis eines polymerisierbaren, ethylenisch ungesättigten Monomeren in Frage, die dem Fachmann für diesen Einsatzzweck geläufig sind. Zu den mit Erfolg einsetzbaren polymerisierbaren Monomeren gehören vorzugsweise solche mit acrylischen und/oder methacrylischen Gruppen.

Insbesondere handelt es sich hierbei u. a. um Ester der α-Cyanoacrylsäure, (Meth)acrylsäure, Urethan(meth)acrylsäure, Crotonsäure, Zimtsäure, Sorbinsäure, Maleinsäure und Itaconsäure mit ein- oder zweiwertigen Alkoholen; (Meth)acrylamide wie z. B. N-isobutylacrylamid; Vinylester von Carbonsäuren wie z. B. Vinylacetat; Vinylether wie z. B. Butylvinylether; Mono-N-vinyl-Verbindungen wie N-Vinylpyrrolidon; und Styrol sowie seine Derivate. Besonders bevorzugt sind die nachfolgend aufgeführten mono- und polyfunktionellen (Meth)acrylsäureester und Urethan(meth)acrylsäureester.
(a) Monofunktionelle (Meth)acrylate
   Methyl(meth)acrylat, N- oder i-Propyl(meth)acrylat, n-, i- oder tert.-Butyl(meth)acrylat und 2-Hydroxyethyl(meth)acrylat.
(b) Difunktionelle (Meth)acrylate
   Verbindungen der allgemeinen Formel: 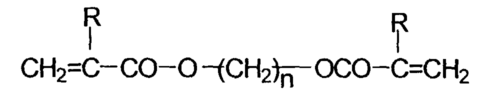 worin R Wasserstoff oder Methyl ist und n eine positive ganze Zahl zwischen 3 und 20, wie z. B. Di(meth)acrylat des Propandiols, Butandiols, Hexandiols, Octandiols, Nonandiols, Decandiols und Eicosandiols, Verbindungen der allgemeinen Formel: 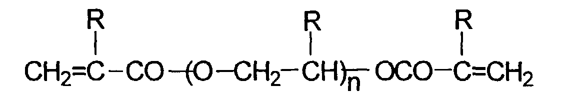 worin R Wasserstoff oder Methyl ist und n eine positive ganze Zahl zwischen 1 und 14, wie z. B. Di(meth)acrylat des Ethylenglycols, Diethylenglycols, Triethylenglycols, Tetraethylenglycols, Dodecaethylenglycols, Tetradecaethylenglycols, Propylenglycols, Dipropylglycols und Tetradecapropylenglycols; und Glycerindi(meth)acrylat, 2,2'-Bis[p-(γ-methacryloxy-β-hydroxypropoxy)phenylpropan] oder Bis-GMA, Biphenol-A-dimethacrylat, Neopentylglycoldi(meth)acrylat, 2,2'-Di(4-methacryloxypolyethoxyphenyl)propan mit 2 bis 10 Ethoxygruppen pro Molekül und 1,2-Bis(3-methacryloxy-2-hydroxypropoxy)butan.
(c) Tri- oder mehrfachfunktionelle (Meth)acrylate
   Trimethylolpropantri(meth)acrylate und Pentaerythritoltetra(meth)acrylat.
(d) Urethan(meth)acrylat
   Umsetzungsprodukte von 2 Mol hydroxylgruppenhaltigen (Meth)acrylatmonomer mit einem Mol Diisocyanat und Umsetzungsprodukte eines zwei NCO Endgruppen aufweisenden Urethanprepolymers mit einem methacrylischen Monomer, das eine Hydroxylgruppe aufweist, wie sie z. B. durch die allgemeine Formel wiedergegeben werden: 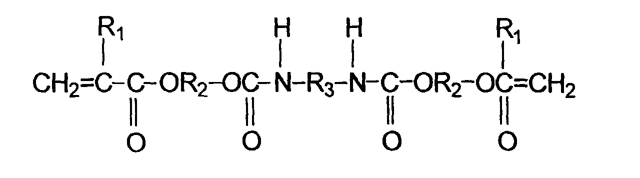 worin R Wasserstoff oder eine Methylgruppe bedeutet, R₂ eine Alkylengruppe und R₃ einen organischen Rest verkörpert.

Die genannten Monomeren werden entweder allein oder in Form einer Mischung von mehreren Monomeren verwendet.

Zu ganz besonders vorteilhaft im erfindungsgemäßen Dentalwerkstoff eingesetzten Monomeren gehören vor allem 2,2-Bis-4(3-methacryloxy-2-hydroxypropoxy)-phenylpropan (Bis-GMA), 3,6-Dioxaoctamethylendimethacrylat (TEDMA), und/oder 7,7,9-Trimethyl-4,13-dioxo-3,14-dioxa-5,12-diaza-hexadecan-1,16-dioxy-dimethacrylat (UDMA).

Der Dentalwerkstoff kann je nach Art des verwendeten Katalysators heiß, kalt und/oder durch Licht polymerisierbar sein. Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis(isobutyroethylester), Benzpinakol und 2,2'-Dimethylbenzpinakol sind geeignet

Als Katalysatoren für die Photopolymerisation können z. B. Benzophenon und seine Derivate sowie Benzoin und seine Derivate verwendet werden. Weitere bevorzugte Photosensibilisatoren sind α-Diketone wie 9,10-Phenanthrenchinon, Diacetyl, Furil, Anisil, 4,4'-Dichlorbenzil und 4,4'-Dialkoxybenzil, Campherchinon wird besonders bevorzugt verwendet. Die Verwendung der Photosensibilisatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester.

Als Katalysatoren für die Kaltpolymerisation werden Radikale liefernde Systeme, z. B. Benzoyl- bzw. Lauroylperoxid zusammen mit Aminen wie N,N-Dimethyl-sym.-xylidin oder N,N-Dimethyl-p-toluidin verwendet. Es können auch dual härtende Systeme zur Katalyse verwendet werden, z. B. Photoinitiatoren mit Aminen und Peroxiden. Als Photokatalysatoren kommen auch Mischungen aus UV-lichthärtenden und im Bereich des sichtbaren Lichts härtenden Katalysatoren in Betracht.

Die Menge dieser Katalysatoren im Dentalwerkstoff liegt üblicherweise zwischen 0,01 bis 5 Gew.-%.

Vorzugsweise dient der erfindungsgemäße Dentalwerkstoff als Zahnfüllungsmaterial. Zahnfüllungsmaterialien werden auch als Zweikomponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste z. B. Benzoylperoxid und in die andere Paste z. B. N,N-Dimethyl-p-toluidin eingearbeitet. Durch Vermischen etwa gleicher Teile der beiden Pasten erhält man ein Zahnfüllungsmaterial, welches in wenigen Minuten aushärtet.

Wenn man bei den letztgenannten Materialien das Amin wegläßt und als Katalysator z. B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird im Mund des Patienten von der Kavität ein Abdruck genommen und ein Gipsmodell hergestellt. In die Kavität des Gipsmodells wird die Paste eingebracht und das Ganze wird in einem Drucktopf unter Hitze polymerisiert. Das Inlay wird entnommen, bearbeitet und dann im Munde des Patienten in die Kavität einzementiert.

Als Füllstoff (A) kommen für die vorliegende Erfindung im Prinzip alle dem Fachmann bekannten mikro- und/oder mesoporen aufweisenden zeolithischen Materialien, insbesondere Zeolithe auf Basis von Kieselsäuren und/oder Aluminosilikaten, Aluminophosphate, Silikoaluminophosphate, metallsubstituierte Aluminophosphate, Metallphosphate wie z. B. Zirkonphosphat, in Frage. Die Füllstoffe (A) sind käuflich erwerblich oder durch literaturbekannte Verfahren herstellbar. Die Synthese der Zeolithe mit Teilchengrößen zwischen 0,5 - 2 µm wird beschrieben in: Breck, D. W., Zeolite Molecular Sieves, Wiley, New York (1974); Szostak, R., Molecular Sieves - Principles of Synthesis and Identification, Van Nostrand Reinhold, New York (1989); Occelli, M. L.; Kessler, H. (Eds), Synthesis of Porous Materials - Zeolites, Clays and Nanostructures, Marcel Dekker, New York (1997). Kolloidale Zeolithe mit Teilchengrößen zwischen 50 - 500 nm sind durch Verfahren, wie in Mintova et al. 1997, 11 ,149; Lovallo et al. 1996, 8, 1579; Schoeman et al. submitted to Microporous Materials; Anderson et al. Access in Nanoporous Materials in Plenum Press, New York 1995, p. 29; Schmidt et al. Microporous Mater. 1995, 5, 1 angegeben, herstellbar.

Vorzugsweise werden als Füllstoffe Zeolithe, wie Faujasit NaX oder NaY sowie deren acide Form HY eingesetzt. Des weiteren werden vorzugsweise dealuminierte Faujasite eingesetzt. Verfahren zur Dealuminierung werden beschrieben in: Anderson, M. V. et al. in J. Chem. Soc. Faraday Trans. I, 1985, 82, p. 1449 und Li, H.-X. et al. in J. Mater. Chem. 1991, 1, p. 79. Zeolith Beta, L oder Silikalit/ZSM-5 sind ebenfalls vorzugsweise einzusetzen. Diese Zeolithe werden mit folgenden Formeln zusammengefaßt:

Faujasit NaX, NaY, HY Naₙ[AlₙSi₁₉₂₋ₙO₃₈₄] x 240 H₂O

n = 48-76 = Y oder Si/Al = 1.5-3 = Y
n = 77-96 = X oder Si/Al = 1-1.5 = X

Beta Naₙ[AlₙSi₆₄₋ₙO₁₂₈] n < 7

L K₆Na₃ [Al₉Si₂₇O₇₂] x 21 H₂O n < 27

ZSM-5 / Silikalit Naₙ[AlₙSi₉₆₋ₙO₁₉₂] x 16 H₂O

An mesoporösen Füllern werden vorzugsweise rein silikathaltige MCM-41 und MCM-48 eingesetzt, deren Synthese von Anderson et al. Access in Nanoporous Materials in Plenum Press, New York 1995, p. 29 und Schmidt et al. Microporous Mater. 1995, 5, 1 beschrieben wird.

Mikroporöse Zeolithe wurden bereits als Füllstoffkomponente in Kompositen beschrieben, ohne jedoch die Porösität durch spezielle Syntheseschritte für den Verbund zwischen organischer und anorganischer Phase auszunutzen. (Wen, J.; Mark, J. E., J. Mater. Sci. 1994, 29, 499. Frisch, H. L. Xue, Y., J. Polym. Sci.: Part A: Polym. Chem. 1995, 33, 1979. Frisch, H. L.; Maaref, S.; Xue, Y.; Beaucage, G.; Pu, Z.; Mark, J. E., J. Polym. Sci. Polym. Chem. Ed. 1996, 34, 673. Kowalek, S.; Pawloswka, M., Szuba, D., J. Mater. Sci. Lett. 1993, 12, 661). Die Beladung der Poren in mesoporösen MCM-41 Füllern mit Monomeren wurde weiterhin dokumentiert. (P. L. Llewellyn, U. Ciesla, H. Decher, R. Stadler, F. Schueth und K. K. Unger, Stud. Surf. Sci. Catal., 1994, 84, 2013. Ng, S. M.; Ogino, S.; Aida, T.; Koyano, K. A.; Tatsumi, T., Macromol. Rapid Commun. 1997, 18, 991).

Als weitere Füllstoffe (B) kommen alle dem Fachmann geläufigen Füllstoffe zur Verbesserung der Qualität des Dentalwerkstoffs (Röntgenopazität, Viskosität, Polierbarkeit, Transparenz, Festigkeit, Brechungsindex) in Frage. Zur Erzielung einer erhöhten Röntgenopazität können Füllstoffe eingesetzt werden, welche z. B. in der DE-OS 35 02 594 beschrieben sind, wobei deren mittlere Primärteilchengröße 5,0 µm nicht übersteigen sollte. Gegebenenfalls können zur Einstellung der Viskosität geringe Mengen an mikrofeiner, pyrogener oder naßgefällter Kieselsäure als Füllstoff (B) in den Dentalwerkstoff eingearbeitet werden.

Als weitere Füllstoffe (B) kommen in Frage: Apatite gemäß der EP 0 832 636 und/oder Partikel gemäß der DE 195 08 586 und/oder DE 41 23 946.

Unter weiteren geeigneten Molekülen, welche durch Silankopplung in die Poren des zeolithischen Füllstoffs (A) eingefügt werden können, sind im Rahmen der Erfindung alle die Möleküle zu verstehen, welche dem Fachmann zur Modifizierung der inneren Oberfläche der Füllstoffe (A) nahegelegt sind. Im Allgemeinen eignen sich die Moleküle, mit welchen auch die äußere Oberfläche des Füllstoffs (A) zur a) Erhöhung der mechanischen Stabilität und Hydrophobizität, b) Verbesserung der Kopplung des anorganischen Füllers an die organische Matrix, c) Bekleidung der Poren mit röntgenopaken Materialien verbessert werden kann (siehe weiter unten).

Man geht dabei so vor, daß vor der Bereitung des Füllstoffs (A) eine bestimmte Menge an geeigneten Molekülen, welche befähigt sind, sich in den Kristallverbund des sich bildenden Füllstoffs (A) eingefügt zu werden, vorhanden sind. Beim Kristallaufbau werden diese geeigneten Moleküle somit in das Kristallgitter chemisch eingebaut, mit der Maßgabe, daß ein Ende des Moleküls in die Pore des Füllstoffs (A) hineinragt. Diese Enden entfalten dann in den Poren die durch Ihre Struktur vorgegebene vorteilhafte Wirkung. Sie hydrophobisieren beispielsweise das Kristallinnere, wodurch eine leichtere und effizientere Befüllung mit hydrophoben Monomeren erfolgen kann. Sie können auch Ankerstellen für polymerisierbare Monomere darstellen, wenn sie selbst polymerisierbare Enden besitzen. So erhält man zusätzlich zur physikalischen Vernetzung der Polymermatrix mit dem Füllstoff (A) eine chemische, was zu einer weiteren Festigkeit der Dentalmischung Anlaß gibt.

Die Mikro- und/oder Mesoporen aufweisenden zeolithischen Füllstoffe (A) werden vor ihrem Einsatz in den Dentalmassen mit polymerisierbaren, ethylenisch ungesättigten oben beschriebenen Monomeren und ggf. den ebenfalls oben beschriebenen Katalysatoren beladen.

Die Füllstoffe (A) können wahlweise befüllt mit Monomeren in die Dentalmasse eingebaut werden oder die Monomere, welche sich in den Poren des Füllstoffes befinden, können zuerst polymerisiert werden, und der Polymer-enthaltende Füllstoff (A) kann anschließend in die Dentalmasse eingebaut werden.

Zusätzlich lassen sich die Poren des Füllstoffs (A) zur Aufnahme von weiteren vorteilhaften Verbindungen nutzen.

Z. B. kann dieser vor der Beladung mit den oben beschriebenen Polymeren mit röntgenopaken Ionen beladen werden. Hier kommen im Prinzip alle dem Fachmann für diesen Zweck geläufigem Ionen in Frage. Hochabsorbierende Ionen wie Cesium, Rubidium, Lanthan, Cerium oder Barium können durch Ionenaustausch in die Poren der mikroporösen Zeolithe eingebracht werden. Entsprechende Verfahren werden beschrieben in Theng, B. K. G.; Vansant, E.; Uytterhoeven, J. B., Trans. Farad. Soc. 1968, 64, p. 3370. Barrer, R. M.; Davis, J. A.; Rees, L. C. L., J. Inorg. Nucl. Chem. 1968, 30, p. 3333. Carvajal, R.; Chu, P.-J.; Lunsford, J. H., J. Catal. 1990, 125, p. 123. Die Beladung mit Cesium oder Lanthan in Alumina-haltigen mesoporösen Trägern erfolgt ebenfalls über Ionenaustausch oder durch Imprägnierung mit gebräuchlichen Salzen. (Kloestra, K. R.; van Bekkum, H., "Progress in Zeolite and Microporous Materials", Stud. Sci. Catal., Vol. 105, 1997, 431. Kloestra, K. R.; van den Broek, J.; van Bekkum, H., Catal. Lett. 1997, 47, 235). Die porösen Träger können dazu entweder mit schweren Elementen (z. B. Ce, In, Ba) in Form von Acetylacetonat Verbindungen oder in Form von Nitraten imbibiert oder mit den Ionen der entsprechenden Salze ausgetauscht werden. Anschließendes Kalzinieren in Luft ermöglicht die Bildung von Oxiden. Ähnliche Verfahren für TiO₂, CeO₂ und BaO sind beschrieben in: Augustynski, J. Electrochimica Acta 1993, 38, 43; Yagi, F.; Kanuka, N.; Tsuji, H.; Kita, H.; Hattori, H., Studies in Surface Science and Catalysis, Vol. 90, 1994, 349; Xie, S.; Mestl, G.; Rosynek, M. P.; Lunsford, J., J. Am. Chem. Soc. 1997, 119, 10186.

Alternativ können die inneren Oberflächen der rein silikathaltigen MCM-Füller belegt werden mit Zinn durch die Verwendung von nicht-wäßrigen Adsorptionsverfahren, wie beschrieben in Ryoo, R.; Jun, S.; Kim, J. M.; Kim, M. J. Chem. Commun. 1997, p. 2225.

Alternativ oder zusätzlich kann der Füllstoff (A) des erfindungsgemäßen Dentalwerkstoffs auch durch gemeinsame Fällung von röntgenopaken Verbindungen in den Poren beladen werden. Die gemeinsame Fällung meint, daß bei der Bereitung des kristallinen Füllstoffs (A) röntgenopake Ionen vorhanden sind, so daß diese in den Kristallverbund und/oder in den Poren des sich bildenden Kristalls eingebaut werden.

Als röntgenopake Verbindung kann weiterhin z. B. das medizinisch getestete BaSO₄ verwendet werden. In einem Verfahren zur Fällung derartiger Verbindungen können zum Beispiel die Zeolithe mit BaCl₂ imprägniert und anschließend getrocknet werden. Die Fällung in Form von BaSO₄ erfolgt durch eine zweite Imprägnierung mit Na₂SO₄. Das dabei entstehende Natriumsalz kann durch Waschen entfernt werden. Die Fällung kolloidaler BaSO₄ Partikel wird beschrieben in Pozarnsky, G.; Matijevic, E., J. Adhersion 1997, 63, 53. Wahlweise können auch BaSO₄ Kolloide dem Dentalwerkstoff beigesetzt werden.

Gleichsam können bacterizide Mittel in dem Füllstoff implementiert werden. Als bacterizide Mittel sieht man insbesondere Hesperedin, Naringenin, Quercetin, Anisic Acid, Acetyl Coumarin, Sitosterol, Caryophyllen und Caryophyllenoxid an. Diese Verbindungen können nach den in US 4,925,660 beschriebenen Verfahren in die Poren des Füllstoffs (A) eingebaut werden.

Bevorzugt kann der mikro- und oder/mesoporöse zeolithische Füllstoff (A) sowie der Füllstoff (B) vor der Anwendung im Dentalwerkstoff optional mit dem Fachmann geläufigen Oberflächenmaterialien modifiziert werden. Diese dienen u. a. zur a) Erhöhung der mechanischen Stabilität und Hydrophobizität, b) Verbesserung der Kopplung des anorganischen Füllers an die organische Matrix, c) Bekleidung der Poren mit röntgenopaken Materialien. In besonderer Ausführungsform ist der Füllstoff mit Silanen der allgemeinen Formel RSi(OX)₃, worin R eine Alkylgruppe mit 1 bis 18 C-Atomen und X eine Alkylgruppe mit 1 oder 2 C-Atomen ist, und/oder Metalloxiden nachbeschichtet. Zur Erhöhung der Stabilität und Hydrophobizität wird insbesondere Trimethylchlorosilan verwendet, wie beschrieben in Koyano, K. A.; Tatsumi, T.; Tanaka, Y.; Nakata, S. J. Phys. Chem. B 1997, 101, p. 9436 und Zhao, X. S.; Lu, G. Q., J. Phys. Chem. B 1998, 102, p. 1156. Wenn zur Nachbeschichtung ein Silan verwendet wird, ist es zweckmäßig, wenn dies in einer Menge von etwa 0,02 bis 2 Gewichtsprozent des Silans, berechnet als SiO₂, angewendet wird, bezogen auf das Gewicht des Füllstoffs (A) bzw. (B). Werden Metalloxide zur Nachbeschichtung eingesetzt, dann geschieht dies in einer Menge von 1 bis 100 Gewichtsprozent, bevorzugt 10 Gewichtsprozent, bezogen auf den Metalloxidgehalt der nicht nachbeschichteten Füllstoffe (A) bzw. (B).

Zu besonders vorteiligen Nachbeschichtungsagenzien gehören unter anderem (CH₃)₃SiCl, Methyltriethoxysilan, Ethyltriethoxysilan, Octyltriethyoxysilan, Octadecyltriethoxysilan, Mono- oder Polyfluoralkylethoxysilan oder auch Silane mit funktionalisierten Organogruppen, die eine spätere weitere Modifizierung durch kovalente Bindungsknüpfung in bekannter Weise ermöglichen. Im letzteren Fall sind solche Organotrialkoxysilane im Hinblick auf den erfindungsgemäßen Einsatz der Partikel als Füllstoffe in polymeren oder polymerisierbaren Systemen bevorzugt, die solche funktionelle Gruppen aufweisen, mit denen sich eine kovalente Einbindung in das Polymermaterial erreichen läßt. Beispiele hierfür sind Trimethoxyvinylsilan, H₂C=C(CH₃)CO₂(CH₂)₃Si(OCH₃)₃, Triethoxyvinylsilan und 3-Glycidoxypropyltrimethoxysilan, sowie Silane mit Hydroxyl-, Carboxyl-, Epoxy- und Carbonsäureestergruppen tragenden anorganischen Resten. Die Einbindung der solcher Art modifizierten Partikel in den Dentalwerkstoff geschieht dabei durch Einarbeitung der Partikel in den Dentalwerkstoff und anschließende Polymerisation bei der eigentlichen Aushärtung des Dentalwerkstoffes. Weitere Metalloxide, welche bevorzugt eingesetzt werden, sind TiO₂, Fe₂O₃ und/oder ZrO₂.

Zweckmäßig ist auch eine Ausführungsform, in der der Füllstoff (A) bzw. (B) zusätzlich mit einer Schicht aus einem polymerisierbaren organischen Bindemittel überzogen ist, welches auf mono- oder mehrfachfunktionellen (Meth)acrylaten und/oder Reaktionsprodukten aus Isocyanaten und OH-gruppenhaltigen Methacrylaten beruht, wobei die Primärteilchengröße der mit polymerisierbarer Bindemittelschicht versehenen Partikel zwischen ca. 0,04 und 1,5 µm liegt, während die Schichtdicke der Überzugsschicht im Bereich von 5 nm bis 50 nm und der Brechungsindex der beschichteten Partikel im Bereich von 1,40 - 1,52 liegt.

Gegenstand der Erfindung ist ebenfalls ein Verfahren zur Herstellung des erfindungsgemäßen Dentalwerkstoffs, welches sich dadurch auszeichnet, daß man den Füllstoff (A) vor der Zubereitung des Dentalwerkstoffs mit polymerisierbaren, ethylenisch ungesättigten Monomeren imprägniert.

Zusätzlich kann der Füllstoff (A) durch Silankopplung mit weiteren geeigneten Molekülen modifiziert werden.

Bevorzugt ist unter anderem die Ausführungsform, bei der man die in dem Füllstoff (A) vor der Zubereitung des Dentalwerkstoffs befindlichen polymerisierbaren, ethylenisch ungesättigten Monomere in deren polymerisierte Form überführt.

Der Füllstoff (A) kann vor der Zubereitung des Dentalwerkstoffs mit gasförmigen polymerisierbaren, ethylenisch ungesättigten Monomeren beladen werden.

Alternativ kann dieser jedoch auch vor der Zubereitung des Dentalwerkstoffs mit flüssigen polymerisierbaren, ethylenisch ungesättigten Monomeren imprägniert werden.

In einem Verfahren zur Herstellung des oben spezifizierten Dentalwerkstoffs wird der anorganische zeolithische Füllstoff (A) vor der Zubereitung des Dentalwerkstoffs mit polymerisierbaren, ethylenisch ungesättigten Monomeren beladen. In einer bevorzugten Ausführungsform wird das Monomer in dem so beladenen Füllstoff vor der Zubereitung des Dentalwerkstoffs in deren polymerisierte Form überführt. Alternativ kann der beladene Füllstoff (A) aber auch erst in den Dentalwerkstoff eingearbeitet und das Monomer im Füllstoff (A) anschließend in diesem mit dem vorhandenen Bindemittel mitpolymerisiert werden.

Erfindungsgemäß erfolgt die Beladung des Füllstoffs (A) mit gasförmigem polymerisierbaren, ethylenisch ungesättigten Monomeren. In einer anderen Ausführungsform belädt man den Füllstoff (A) vor der Zubereitung des Dentalwerkstoffs jedoch mit flüssigen polymerisierbaren, ethylenisch ungesättigten Monomeren.

Das Beladen des Füllstoffs (A) erfolgt also bevorzugt durch Behandlung des mikro- und/oder mesoporösen zeolithischen Füllstoffs (A) mit einem Dampf oder Gas sowie alternativ mit flüssigen polymerisierbaren, ethylenisch ungesättigten oben beschriebenen Monomeren und ggf. den ebenfalls oben beschriebenen Katalysatoren. Bei beiden Vorgängen füllen sich die Poren des Materials (A) mit den Monomeren und durchdringen deren gesamtes festes Porengefüge. Bei der folgenden Polymerisation polymerisiert das in den Poren befindliche Monomer durch. Die Enden der Polymerstränge ragen dabei aus den Poren des Füllstoffs (A) heraus. Diese Enden nun werden mit dem Bindemittel des Dentalwerkstoffs durch Polymerisationsreaktionen weiterverbunden. Durch das sich im Füllstoff (A) ausgebildete polymere Netzwerk, welches exoseitig mit dem Bindemittel des Dentalwerkstoffs chemisch verbunden ist, wird gewährleistet, daß sich eine überaus starke physikalische Bindung zwischen dem Füllstoff (A) und dem Bindemittel des Dentalwerkstoffs ausbildet, welche durch Hydrolyse nicht mehr zerstört werden kann. Gleichsam erhält man einen Dentalwerkstoff, der sich durch eine besondere Festigkeit auszeichnet, die von Dentalwerkstoffen des Standes der Technik nicht erreicht wird. Auch wird damit die Spaltung zwischen den organischen Partikeln und der organischen Matrix fast gänzlich unterdrückt. Dies steigert die Haltbarkeit der erfindungsgemäßen Dentalmassen.

Ein weiterer Aspekt der vorliegenden Erfindung ist die Verwendung von mit polymerisierbaren, ethylenisch ungesättigten Monomeren oder durch Silankopplung mit weiteren geeigneten Molekülen beladenem zeolithische Füllstoff (A) in erfindungsgemäßen Dentalwerkstoffen.

### Beispiele:

### 1) Herstellung der Füllstoffe (A)

### 1a) Zeolithfüller

- Faujasit NaY bzw. dessen acide Form (PQ Corporation)
- Faujasit NaX (UOP)
- Mordenit (UOP)
- H-ZSM-5 (UOP)

Die Dealuminierung der aluminiumhaltigen Precursor der Zeolithe erfolgt durch deren Reaktion in einem Strom von SiCl₄-Dampf bei 550 °C für sechs Stunden. Die Herstellung der kolloidalen Silikate, Zeolith L bzw. Beta erfolgt nach Lit.-methoden (Mintova et al. 1997, 11, 149; Lovallo et al. 1996, 8, 1579; Schoeman et al. submitted to Microporous Materials).

Alle Zeolithe wurden vor ihrem Einsatz im Sauerstoffstrom bei einer Aufheizrate von 2 °C/min bis zu 400 °C calciniert.

### 1b) Mesoporöse Träger

- MCM-41
- MCM-48

Die Herstellung von MCM-41 erfolgt nach Anderson et al. (Access in Nanoporous Materials in Plenum Press, New York 1995, p. 29) die von MCM-48 nach Schmidt et al. (Microporous Mater. 1995, 5, 1).

### 1c) Funktionalisierte mesoporöse Träger

Die Synthese der funktionalisierten mesoporösen Träger MA-MCM erfolgt durch eine Abwandlung in der Synthese des rein silikathaltigen MCM-41:

MCM-41: 13.64 g Wasser und 4.90 g Methanol werden vermischt und mit 0.13 ml 50 Gew.% NaOH auf einen pH von 12.7 eingestellt. 1.408 g Cetyltrimethyl Ammoniumchlorid werden unter Rühren hinzugefügt. Anschließend werden 1.25 ml Tetramethoxysilan (TMOS) beigegeben. Der Niederschlag wird 3 bis 24 Stunden bei Raumtemperatur belassen, anschließend gefiltert und bei 70 °C getrocknet. Das Templat wird durch Heizen im Stickstoffstrom bei 550 °C mit einer Heizrate von 2 °C/Minute entfernt. Der Stickstoffstrom wird dabei nach 6 Stunden gegen Luft ausgewechselt und bei gleicher Temperatur für weitere 2 Stunden gehalten.

MA-MCM: Die Synthese erfolgt wie oben beschrieben. Die 1.25 ml TMOS aus der MCM-41 Synthese werden in der MA-MCM Synthese teilweise ersetzt durch 3-Trimethoxysilylpropylmethacrylat (TMSiPMA). Der Anteil an TMSiPMA kann dabei um folgende stöchiometrische Anteile variiert werden: TMOS : TMSiPMA = 7 : 1, 5 : 1 oder 3 : 1. Das Templat wird in diesem Fall durch Extraktion mittels 1 M Salzsäure in Äthanol entfernt. Dazu werden ca. 2 g MA-MCM in ca. 50 ml HCl/Äthanol für 2 Stunden im Rückfluß gekocht.

### 2) Befüllen der mikro- und mesoporösen zeolithischen Träger

### 2a) Gasphasenabsorption

1 g des mikro- bzw. mesoporösen zeolithischen Materials wird im Hochvakuum von weniger als 3x10⁻⁴ Torr bei 200-300 °C für 12 h belassen. In einer zweiten Apparatur wird MMA (98 %, Aldrich) entgast durch dreimaliges Frieren mit flüssigem Stickstoff, Evakuieren und Tauen. Bei RT werden die Materialien dann dem MMA-Dampf/Gas 2 h lang unter Rühren ausgesetzt. Die MMA gefüllten Materialien werden dann in eine unter Stickstoff gehaltene Box gebracht und mit 10 mg Benzoylperoxid (99 %, Aldrich) gemischt. Dann wird das Material vorpolymerisiert durch Erhitzen auf 70 °C für 1-2 h. Sodann wird das Material für 2 h auf 120 °C erwärmt und abschließend für 18 h im Vakuum endpolymerisiert und von möglichen verbleibenden Monomeren befreit. Das Verschwinden der C=C-Doppelbindungsschwingung im FTIR deutet auf die erfolgte Polymerisation hin. Stickstoffadsorptions/desorptions-messungen belegen eine BET-Oberfläche, die stark reduziert ist gegenüber dem nicht beladenen Material. Thermogravimetriedaten zeigen weiterhin eine stärkere Beladung des Materials mit zunehmendem Porenvolumen an. Die thermogravimetrischen Daten sind in Tabelle 1 eingearbeitet.

Die Ergebnisse der Messungen sind in Tabelle 1 aufgelistet.

**Tabelle 1**

| Material | Porengröße [Å] | Porenvolumen | | Gew.-% PMMA | g PMMA/g Material |
|---|---|---|---|---|---|
| | | vor (ml/g) | nach Befüllung | | |
| NaY/X | 7.4 | 0.32 | 0 | 22 | 0.3 |
| Beta | 7.6 X 6.4 | 0.32 | - | 10.4 | 0.12 |
| MOR | 6.7 x 7.0 | 0.13 | 0 | 11 | 0.12 |
| ZSM-5 | 5.3 X 5.6 | 0.1 | 0.07 | 7.5 | 0.07 |
| | 5.1 X 5.5 | | | | |
| silica | 15-150 | - | - | 5 | 0.07 |
| MCM-41 | ca. 28 | 0.62 | 0.62 | 27 | 0.39 |
| MCM-48 | ca. 30 | 0.92 | 0 | 45 | 0.82 |

### 2b) Flüssigphasenabsorption

5-10 g des porösen Materials werden im Luftstrom bei 200-300 °C kalziniert und anschließend im Stickstoffstrom 2 Stunden dehydratisiert. Die Monomere Methylmethacrylat, Tetra- oder Trisethylenglykoldimethacrylat werden von vorhandenem Inhibitor befreit und wie oben für MMA beschrieben gereinigt. Das mit 0.5 Gew.% Benzoylperoxid oder Campherchinon vermischte Monomer wird unter Stickstoffatmosphäre zu dem trockenen porösen Material getropft, bis eine Art Paste entsteht. Die Polymerisation erfolgt thermisch (bei Verwendung von Benzoylperoxid) oder durch Bestrahlung mit UV-Licht bei 400-500 nm (bei Verwendung von Campherchinon).

Die im folgenden aufgeführten Füller wurden auf diese Weise mit Monomer versetzt. Der resultierende Polymergehalt wird mittels Thermogravimetrie bestimmt. Der Gewichtsanteil an Monomer, der benötigt wird, um die Poren zu beladen und einen pastenartigen Füller zu erstellen, variiert entsprechend des Porenvolumens und der Oberfläche des verwendeten Füllers wie folgt:

| Gewichtsanteil | | |
|---|---|---|
| Monomer Gew.%: | ca. 20 % | bei amorphem Silika |
| | ca. 40 % | bei ZSM-5 |
| | ca. 50-60 % | bei Mordenit und Faujasit |
| | ca. 70 % | bei MCM-41 |
| | ca. 80-90 % | bei MCM-48 |

Stickstoff Adsorptionsmessungen zeigen, daß die Poren der Füller nach Flüssigphasenabsorption vollständig beladen sind. FTIR Spektroskopie bestätigt eine erfolgreiche Polymerisierung in photopolymerisierten wie in thermisch polymerisierten Proben.

Die oben genannten Faujasite wurden entweder in ihrer Natrium-Form verwendet oder durch Ionenaustausch mit Barium beladen.

Es wurden weiterhin MCM-41 Füller in ihrer ursprünglichen Form verwendet oder nach einer Silanisierungsreaktion mit H₂C=C(CH₃)CO₂(CH₂)₃Si(OCH₃)₃, in der polymerisierbare Methacrylatgruppen an die Porenwand angebracht wurden.

Weiterhin wurden die oben beschriebenen neuartigen MA-MCM-41 Füller verwendet.

## Patentansprüche

1. Dentalwerkstoff auf Basis von polymerisierbaren, ethylenisch ungesättigten Monomeren als Bindemittel, eines Katalysators für die Kalt-, Heiß- und/oder Photopolymerisation und bezogen auf den Dentalwerkstoff 1-95 Gew.-% eines anorganischen zeolithischen Füllstoffs (A) sowie bezogen auf den Dentalwerkstoff 0-95 Gew.-% von weiteren Füllstoffen (B),
**dadurch gekennzeichnet,**
daß der zeolithische Füllstoff (A) mit polymerisierbaren, ethylenisch ungesättigten Monomeren oder mit deren polymerisierter Form und/oder durch Silankopplung mit weiteren geeigneten Molekülen beladene Mikro- und/oder Mesoporen aufweist.

2. Dentalwerkstoff nach Anspruch 1,
**dadurch gekennzeichnet,**
daß der Füllstoff (A) ein Zeolith ist und man diesen ggf. vor Befüllung mit Monomeren dealuminiert.

3. Dentalwerkstoff nach Anspruch 1 und/oder 2,
**dadurch gekennzeichnet,**
daß im auspolymerisierten Zustand das Verhältnis der Gew.-Teile von Polymer zu Füllstoff (A) zu Füllstoffen (B) bei Dentalfüllungen im Verhältnisbereich von 10-40 zu 1-20 zu 89-40 liegt (in Summe 100).

4. Dentalwerkstoff nach Anspruch 1 bis 3,
**dadurch gekennzeichnet,**
daß man den Füllstoff (A) zusätzlich mit röntgenopaken Ionen durch Ionenaustausch belädt.

5. Dentalwerkstoff nach Anspruch 1 bis 3,
**dadurch gekennzeichnet,**
daß man den Füllstoff (A) zusätzlich durch gemeinsame Fällung in den Poren und/oder im Kristallgitter mit röntgenopaken Verbindungen belädt.

6. Dentalwerkstoff nach einem der vorhergehenden Ansprüche,
**dadurch gekennzeichnet,**
daß man den Füllstoff (A) zusätzlich mit bacteriziden Mitteln belädt.

7. Dentalwerkstoff nach einem der vorhergehenden Ansprüche,
**dadurch gekennzeichnet,**
daß man die Oberfläche des Füllstoffs (A) und/oder (B) chemisch modifiziert.

8. Verfahren zur Herstellung eines Dentalwerkstoffs nach Anspruch 1,
**dadurch gekennzeichnet,**
daß man den Füllstoff (A) vor der Zubereitung des Dentalwerkstoffs mit polymerisierbaren, ethylenisch ungesättigten Monomeren imprägniert.

9. Verfahren nach Anspruch 8,
**dadurch gekennzeichnet,**
daß man zusätzlich den Füllstoff (A) durch Silankopplung mit weiteren geeigneten Molekülen modifiziert.

10. Verfahren nach Anspruch 8 und/oder 9,
**dadurch gekennzeichnet,**
daß man die in dem Füllstoff (A) vor der Zubereitung des Dentalwerkstoffs befindlichen polymerisierbaren, ethylenisch ungesättigten Monomere in deren polymerisierte Form überführt.

11. Verfahren nach Anspruch 8 bis 10,
**dadurch gekennzeichnet,**
daß man den Füllstoff (A) vor der Zubereitung des Dentalwerkstoffs mit gasförmigen polymerisierbaren, ethylenisch ungesättigten Monomeren belädt.

12. Verfahren nach Anspruch 8 bis 10,
**dadurch gekennzeichnet,**
daß man den Füllstoff (A) vor der Zubereitung des Dentalwerkstoffs mit flüssigen polymerisierbaren, ethylenisch ungesättigten Monomeren belädt.

13. Verwendung von mit polymerisierbaren, ethylenisch ungesättigten Monomeren oder durch Silankopplung mit weiteren geeigneten Molekülen beladenem kristallinen anorganischen zeolithischen Füllstoff (A) in Dentalwerkstoffen nach Anspruch 1.
